# EUROPEAN PATENT APPLICATION

(11) **EP 2 949 359 A1**
(43) Date of publication of application: **02.12.2015**
(21) Application number: 13872485.1
(22) Date of filing: 23.01.2013
(51) Int. Cl.: A61N 1/30

(54) **DRUG ADMINISTRATION DEVICE, AND CONTROL METHOD**

(71) Applicant: Terumo Kabushiki Kaisha, Tokyo 151-0072 (JP)
(72) Inventor: NARIMATSU, Kiyoyuki, Ashigarakami-gun Kanagawa 259-0151 (JP)
(74) Representative: Casalonga
(86) International application number: PCT/JP2013/000328
(87) International publication number: WO 2014/115183

(57) **Abstract**

A drug administration device which applies a voltage between electrodes so as to percutaneously administer a drug measures a current value between the electrodes at predetermined time intervals, calculates an administration rate of the drug, based on the measured current value, and causes a display to display the calculated administration rate.

## Description

### Technical Field

The present invention relates to a drug administration device which percutaneously administers a drug and a control method thereof.

### Background Art

In general, oral administration or injection administration is widely used in order to administer a drug. In a case of oral administration, the drug is conveniently and very safely taken, but there is a problem in that a water-soluble drug or a polymeric drug cannot be sufficiently absorbed. In addition, in a case of injection administration, there is an advantage in that the drug works quickly, but there is an adverse effect in that a patient has to feel pain.

Currently, in order to compensate for the problems or adverse effects in these methods used in the related art, a drug administration system (hereinafter, referred to as a percutaneous drug administration system) through skin has been progressively developed. As the percutaneous drug administration system, several methods such as methods of using electrical energy, methods of using ultrasound waves, or the like have been proposed.

Among the methods of using electrical energy, iontophoresis is known as a partially commercialized method which has been studied for a long time. The iontophoresis is a method in which a charged drug is moved and absorbed in a site of a keratinous layer on the principle of electrophoresis, by placing positive and negative electrodes on two separated points of skin, and by generating a current which crosses the keratinous layer of the skin and then passes through an internal body side from the keratinous layer so that the current is connected from the positive electrode to the negative electrode. In principle, the charged drug is subject to promoted absorption, but the current also causes water to flow therein. For this reason, it has been reported that a drug having no charge or a drug having high molecular weight is also able to have improved skin permeability.

As an example of the iontophoresis, PTL 1 discloses that a patch having an electrode containing a drug is combined with a controller driven by a battery so as to supply power from the controller to the electrode. The controller disclosed in PTL 1 is small since the controller does not need to set a complicated profile and is used simply for switching currents to be supplied. Moreover, since the controller is driven by the battery, there is no possibility of restricting a patient to whom the drug is administered.

In addition, PTL 2 discloses a configuration in which a controller combined with a patch and a dedicated cradle are provided so that the dedicated cradle supplies the controller with a profile (current value setting or the like) to be set depending on the patch, by wireless communication. According to PTL 2, it is possible to easily perform more complicated current value control while maintaining design features of a device.

### Citation List

### Patent Literature

PTL 1: JP-T-11-506368
PTL 2: JP-A-2010-172475

### Summary of Invention

### Technical Problem

Incidentally, according to the portable iontophoresis as disclosed in PTL 1 or PTL 2, a patient, a doctor, a nurse, or the like cannot judge whether or not drug administration is actually in progress in accordance with the set supply current or the set profile. In general, a current amount and a drug administration amount are correlated with each other. In the iontophoresis, the drug administration amount is controlled by adjusting the current between electrodes. Therefore, a constant current source is used in order to cause the set current to flow between the electrodes. However, an applicable voltage is limited since the patient may feel burdened or may encounter an electric shock hazard, for example. If the patient has strong skin resistance, or if the skin resistance is greatly changed due to some reasons, there is a possibility that the set current, that is, a drug administration rate cannot be maintained. However, according to the drug administration device in the related art, a user cannot confirm this change in the drug administration amount while the drug is administered.

The present invention is made in view of the above-described circumstances, and an object thereof is to enable a patient, a health care worker, or the like to easily recognize progress in drug administration using iontophoresis.

### Solution to Problem

In order to achieve the above-described object, a drug administration device according to an aspect of the present invention includes the following configurations. That is, there is provided a drug administration device which applies a voltage between electrodes so as to percutaneously administer a drug by using iontophoresis. The drug administration device includes measurement means for measuring a current value between the electrodes at predetermined time intervals, calculation means for calculating an administration rate of the drug based on the current value measured by the measurement means, and control means for causing a display to display the administration rate calculated by the calculation means.

### Advantageous Effects of Invention

According to the present invention, progress in drug administration using iontophoresis is visible. Therefore, a patient, a health care worker, or the like can easily recognize the progress in drug administration.

Other features and advantages of the present invention will become apparent from the following description with reference to the accompanying drawings.

### Brief Description of Drawings

Fig. 1 is a general perspective view illustrating a percutaneous drug administration system according to a first embodiment.
Fig. 2a is a schematic bottom view of a patch.
Fig. 2b is a general perspective view when the patch is viewed from above.
Fig. 3 is a general perspective view when a controller is viewed from below.
Fig. 4 is a schematic block diagram of the controller.
Figs. 5a and 5b are views illustrating an example where drug administration is performed.
Fig. 6 is a flowchart illustrating a drug administration process using the controller.
Figs. 7a and 7b are views illustrating an example of content displayed on a display of the controller.
Fig. 8 is a view illustrating an example of content displayed on the display of the controller.

### Description of Embodiments

Hereinafter, preferred embodiments according to the present invention will be described with reference to the accompanying drawings.

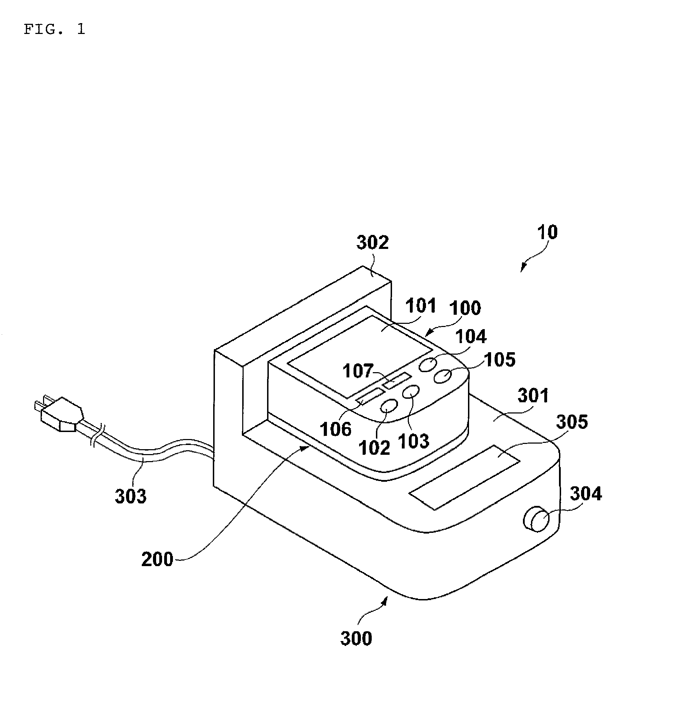

As illustrated in Fig. 1, a percutaneous drug administration system 10 according to a first embodiment is configured to include a patch 200, a controller 100, and a cradle 300 as a basic configuration. The controller 100 and the patch 200 are freely detachable, and are combined with each other (as will be described later) so as to configure a drug administration device.

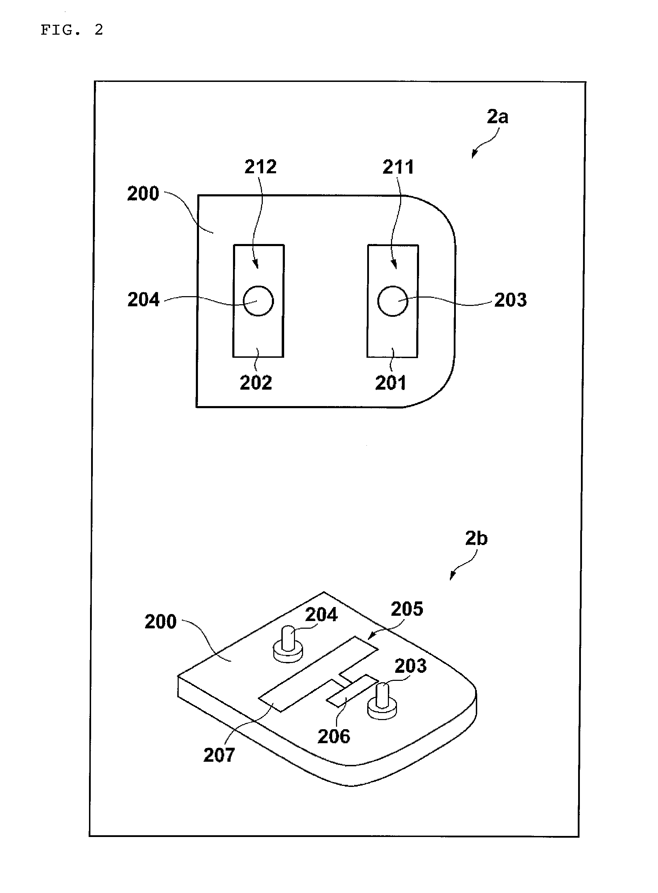

The percutaneous drug administration system 10 realizes iontophoresis in which the drug administration device having the patch 200 and controller 100 integrated with each other sticks to a patient's skin and a current is caused to flow from the controller 100 to the patch 200 so as to percutaneously administer a drug 211 (refer to Fig. 2a) prepared in the patch 200.

The iontophoresis is a method of using electrical energy so as to mainly promote an ionic drug to permeate a biological membrane, and is used in order to promote percutaneous absorption of drugs. The patch 200 has two reservoirs including electrodes (as will be described later), and is sealed with a drug, for example, in such a way that an anionic drug is contained in a cathode vessel and a cationic drug is contained in an anode vessel. The patch 200 sticks to the skin and a voltage with several volts is applied thereto so that a current with several hundred µA flows between the electrodes. In this manner, the drug is transferred to the skin, and concurrently, an endogenous ion making a pair with the drug is extracted into one reservoir from the skin. The other reservoir also induces ion exchange so as to establish an electrical circuit. Furthermore, if the skin is loaded with the voltage, water moves from an anode toward a cathode. According to this movement, the drug which does not dissociate therefrom is also percutaneously delivered.

As illustrated in Figs.2a and 2b, the patch 200 has a donor reservoir 201, a return reservoir 202, a pair of connecting pins 203 and 204, and an RF tag chip 205. As illustrated in Fig.2b, the patch 200 has a thin plate shape, and the pair of connecting pins 203 and 204 are disposed to protrude upward on the upper surface of the patch 200. Here, in order to facilitate understanding of the description, a vertical direction in the percutaneous drug administration system 10 indicates a direction perpendicular to a contact surface between the patch 200 and the cradle 300 which are illustrated in Fig. 1, the controller 100 side indicates an upward direction, and the cradle 300 side indicates a downward direction. However, orientations used in practice are not limited thereto. In addition, both corners at one short side of a rectangular shape in a plan view are chamfered so as to have a round shape. This prevents a user from mistaking an orientation thereof when connecting the patch 200 to the controller 100.

The RF tag chip 205 is installed at a substantially central portion on an upper surface of the patch 200. The RF tag chip 205 is a passive RF tag which does not require an external power source such as a battery or the like, and has an IC chip 206 and an antenna 207. The antenna 207 is formed on the upper surface of the patch 200 by etching aluminum, copper, or silver or by using a printing method, and has a function of receiving a signal from the controller 100 and reflecting the signal. The IC chip 206 and the antenna 207 are covered with a film or the like (not illustrated).

The IC chip 206 is configured to include a high-frequency circuit operated at 800 MHz to 2.54 GHz, a power recovery circuit, a memory control circuit, a memory, and the like (all are not illustrated). A power source of the RF tag chip 205 which is a passive RF tag reproduces DC power by causing the antenna 207 to receive a signal from outside by using the power recovery circuit and converting the signal into power. Since this passive RF tag is used, a power source such as a battery or the like is not required, thereby enabling the patch 200 to be miniaturized. In addition, since no power source is provided, an operable period of the RF tag chip 205 is no longer limited, thereby facilitating management of the patch 200. Without being limited to the passive RF tag, the RF tag chip 205 may employ an active RF tag, for example. Identification information indicating types of contained drug and a parameter indicating a relationship between an administration amount of the contained drug and a current value are recorded on the IC chip 206.

The donor reservoir 201 and the return reservoir 202 are disposed at positions symmetrical with each other from substantially the center on a lower surface (surface in contact with a patient's skin) of the patch 200. The donor reservoir 201 is filled with the drug 211, and has a function as a positive electrode plate. That is, in the present embodiment, the donor reservoir 201 is an anode vessel, and the drug 211 is a drug such as a cationic drug having a positive ion or the like. The return reservoir 202 has the same structure as the donor reservoir 201, but has a function as a negative electrode plate, and forms a cathode vessel. The return reservoir 202 is filled with a physiological salt solution 212 instead of the drug 211. The physiological salt solution 212 has a negative ion. In a case of the patch 200 which administers a drug such as an anionic drug having the negative ion or the like, the cathode vessel serves as the donor reservoir.

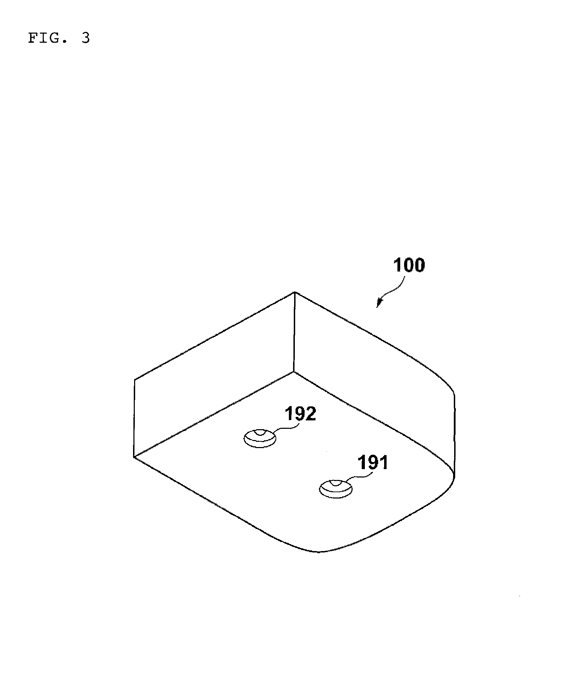

As described above, the connecting pins 203 and 204 are disposed to protrude on the upper surface of the patch 200, and are connected to the donor reservoir 201 and the return reservoir 202 inside the patch 200. In addition, the connecting pins 203 and 204 are inserted into connecting hooks 191 and 192 (refer to Fig. 3) having a concave shape in the controller 100, thereby providing a function of supplying electricity from the controller 100 to the donor reservoir 201 and the return reservoir 202. In addition, the pair of connecting pins 203 and 204 and the pair of connecting hooks 191 and 192 may be disposed at asymmetrical positions from the center on each installation surface. Alternatively, a diameter of the connecting pins 203 and 204 may be different from a diameter of the connecting hooks 191 and 192. This prevents a user from mistaking an orientation thereof when connecting the patch 200 to the controller 100.

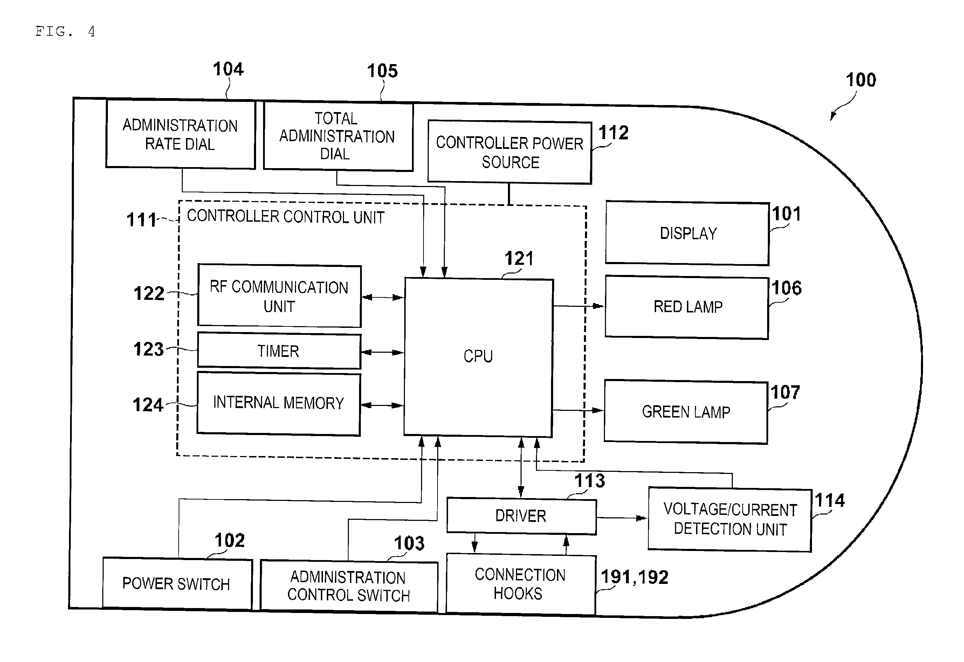

As illustrated in Figs. 1, 3, and 4, the controller 100 according to the present embodiment has the same shape as the patch 200 in a plan view. However, as a matter of course, the shape of the controller 100 or the like is not limited thereto. The controller 100 has a display 101 such as an LCD or the like, the pair of connecting hooks 191 and 192, a power switch 102, an administration control switch 103, a red lamp 106, a green lamp 107, an administration rate dial 104, and a total administration amount dial 105. In addition, the controller 100 internally includes a controller control unit 111 which is a control circuit of the controller 100, a controller power source 112, a driver 113, and a voltage/current detection unit 114.

As illustrated in Fig. 3, the connecting hooks 191 and 192 are holes which have a size and are arranged at positions on the lower surface in the controller 100 so as to enable engagement with the connecting pins 203 and 204. The connecting hooks 191 and 192 engage with the connecting pins 203 and 204, thereby providing a function of supplying power between the controller 100 and the patch 200. The connecting hook 191 is connected to the connecting pin 203, and the connecting hook 192 is connected to the connecting pin 204.

In a state where the power switch 102 is turned on, power is supplied from the controller power source 112 to the controller control unit 111, thereby enabling communication between the controller 100 and the patch 200 (RF tag chip 205), administration of the drug 211, or the like. In addition, in a state where the power switch 102 is turned off, the power is no longer supplied from the controller power source 112 to the controller control unit 111. As a type of the power switch 102, various types such as an alternate operation type, a momentary operation type, and the like are conceivable, but the type is not limited to both of these. Respective switches to be described below are not similarly limited thereto.

If the administration control switch 103 is in a turned-on state, the controller control unit 111 administers the drug 211 into a body of a patient. In a state where the administration control switch 103 is turned off, the controller control unit 111 stops and completes administration of the drug 211. A configuration may be adopted in which if the drug having an administration amount set by the total administration amount dial 105 is administered after the administration control switch 103 is turned on, the controller control unit 111 judges that the administration is completed, completes the administration of the drug 211, and turns off the administration control switch 103.

The red lamp 106 and the green lamp 107 are display lamps using a light emitting diode (LED), for example. The red lamp 106 has a function of notifying a patient, a qualified user such as a doctor and a nurse, or the like of a malfunction of the controller 100 by means of fast blinking. In addition, the red lamp 106 has a function of notifying the patient or the user of an insufficient residual power amount of the controller power source 112 by means of slow blinking. The green light 107 has a function of notifying the patient or the user of normal drug administration by means of blinking. However, uses of the red lamp 106 and the green lamp 107 are not limited thereto. In addition, a configuration may be adopted in which the notification function of the red lamp 106 or the green lamp 107 is realized by the display 101, and the red lamp 106 or the green lamp 107 is omitted.

The controller power source 112 is a secondary battery having a charging function, and may have a form and a size which allow the controller 100 to be portable. As the controller power source 112, a power source having a power storage function such as a dry cell, a button-type battery, and the like may be employed. In this case, the cradle 300 for charging is no longer required.

The controller control unit 111 has a central processing unit (CPU) 121 serving as a main control unit, an RF communication unit 122, a timer 123, and an internal memory 124. The driver 113, the voltage/current detection unit 114 and the like are connected to the controller control unit 111. The above-described respective functional units are realized by the CPU 121 reading a program and executing software processing in cooperation with the RF communication unit 122, the internal memory 124, the driver 113, and the like. The controller control unit 111 performs administration of the drug 211, at an administration rate and during an administration time, all of which are set by the administration rate dial 104 and the total administration amount dial 105. In addition, when the drug 211 is administered, even if an administration rate is changed due to increase of resistance inside a body of a patient, variations in a power source voltage or the like, the controller control unit 111 performs proportional integral differential (PID) control or proportional integral (PI) control for example, so as to maintain a set administration state.

The RF communication unit 122 communicates with the RF tag chip 205 of the patch 200. In the present embodiment, the RF communication unit 122 has a function of acquiring a parameter indicating a relationship between types of the drug 211 or an administration amount and a current from the patch 200 and transmitting the parameter to the CPU 121. The internal memory 124 includes a nonvolatile memory such as a flash memory or the like, and types of drug, parameters, or the like received from the patch 200 via the RF communication unit 122 and the CPU 121 are written in the internal memory 124.

The driver 113 has a function of supplying a current to the connecting hooks 191 and 192, based on control information provided by the controller control unit 111. For example, the driver 113 can convert a supply current freely in time by using a pulse width modulation (PWM) method, and there is no limitation to the type of circuit that can be used for a current supply pattern. The voltage/current detection unit 114 detects a voltage and a current which are applied via the connecting hooks 191 and 192, and provides the CPU 121 with the detection result. This enables the CPU 101 to measure a current value and a voltage value between electrodes. The RF communication unit 122, the internal memory 124, the driver 113, and the voltage/current detection unit 114 may be integrated with the CPU 121.

As illustrated in Fig. 1, the cradle 300 has a shape including a cradle main body 301 and a cradle wall portion 302 which have substantially rectangular parallelepiped shapes. Both corners at a side facing the cradle wall portion 302 having a rectangular shape in a plan view are chamfered so as to have a round shape. This shape is the same as that of the patch 200 and the controller 100, all of which are described above. A user can use this shape as a direction guide when the user integrates the patch 200 and the controller 100 with each other so as to set both of these on the cradle main body 301 as illustrated in Fig. 1.

In addition, the cradle 300 includes a power code 303, a power switch 304, and an operation display unit 305. Drive power is supplied from a wall power source to the cradle 300 by the power code 303 extending from a rear surface. For example, an alternating current (AC) of 100 V or 200 V is used as a power source. Here, an example has been described in which a commercially available general wall power source is used, but the cradle 300 maybe driven by a battery. The power switch 304 is disposed on a side surface of the cradle main body 301, and supplies the power in a turned-on state. The power charges the controller power source 112 of the controller 100 mounted on the cradle 300 as illustrated in Fig. 1. As a charging method, non-contact charging without using a contact may be performed, or charging may be performed by using electrodes. The operation display unit 305 notifies a user of charging in progress, charging completed, or the like.

The percutaneous drug administration system 10 according to the present embodiment is basically configured as described above. Next, a case will be described where the drug 211 is administered to a patient by using the percutaneous drug administration system 10.

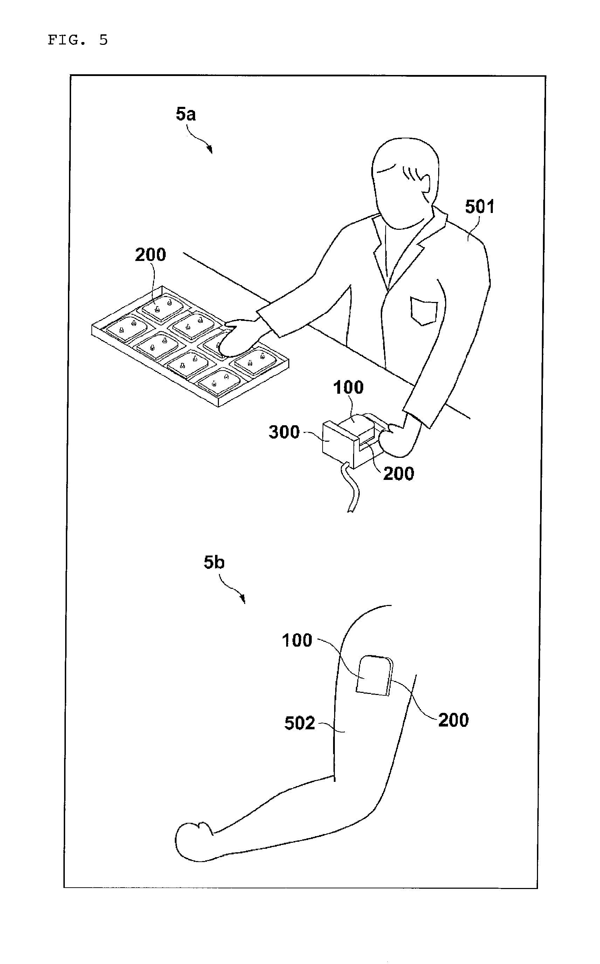

As illustrated by Fig.5a, a user 501 first prepares the patch 200, the controller 100, and the cradle 300 in his or her hand. At a manufacturing stage, the donor reservoir 201 of the patch 200 is filled with the drug 211 in advance, and the return reservoir 202 is filled with the physiological salt solution 212 in advance. The above-described identification information, the parameter indicating the relationship between the administration rate and the current value, or the like is recorded on the IC chip 206 of the patch 200 at the manufacturing stage.

As illustrated by Fig.5a, the user 501 selects the suitable patch 200 from multiple patches, and integrates the patch 200 and the controller 100 with each other by causing the pair of connecting pins 203 and 204 to engage with the pair of connecting hooks 191 and 192. The user 501 or a patient 502 detaches a predetermined protection film from the patch 200, and sticks the patch 200 and the controller 100 on the patient 502 (refer to Fig.5b). The lower surface of the patch 200, that is, the surface in contact with the patient 502 has a moderate adhesive strength. Accordingly, the patch 200 and the controller 100 do not drop off from the patient 502.

The user 501 or the patient 502 turns on the administration control switch 103. This action causes the CPU 121 to read the parameter indicating the relationship between the administration amount and the current amount from the internal memory 124, to determine an amount of the current to be applied so as to satisfy the set administration speed, and to supply power from the driver 113 to the connecting hooks 191 and 192. In this manner, on the principle of electrophoresis, a current is generated which crosses the keratinous layer of the skin of the patient 502 from the donor reservoir 201 serving as a positive electrode, which then passes through the internal body side from the keratinous layer, and which flows to the return reservoir 202 serving as a negative electrode. Accordingly, the drug 211 which is positively charged moves similarly to the above-described current, but is absorbed into the body of the patient 502 through a site of the keratinous layer. Contrary to the above-described current, the negative ion contained in the physiological salt solution 212, for example, such as a chloride ion, crosses the keratinous layer of the patient 502 from the return reservoir 202, then passes through the internal body side from the keratinous layer, and moves to the donor reservoir 201. In addition, the drug 211 is administered to the patient 502 at the set administration rate and during the set administration time.

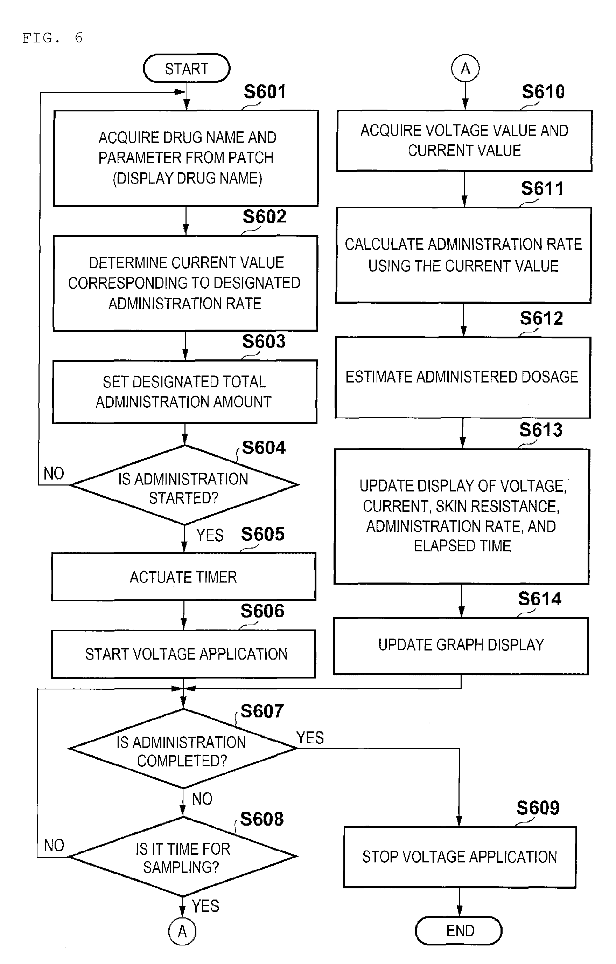

Fig. 6 is a flowchart illustrating an operation of the drug administration device according to the present embodiment. The following process is realized in such a way that the power switch 102 of the controller 100 is turned on, power is supplied to the controller control unit 111, and the CPU 121 executes a predetermined program stored in the internal memory 124.

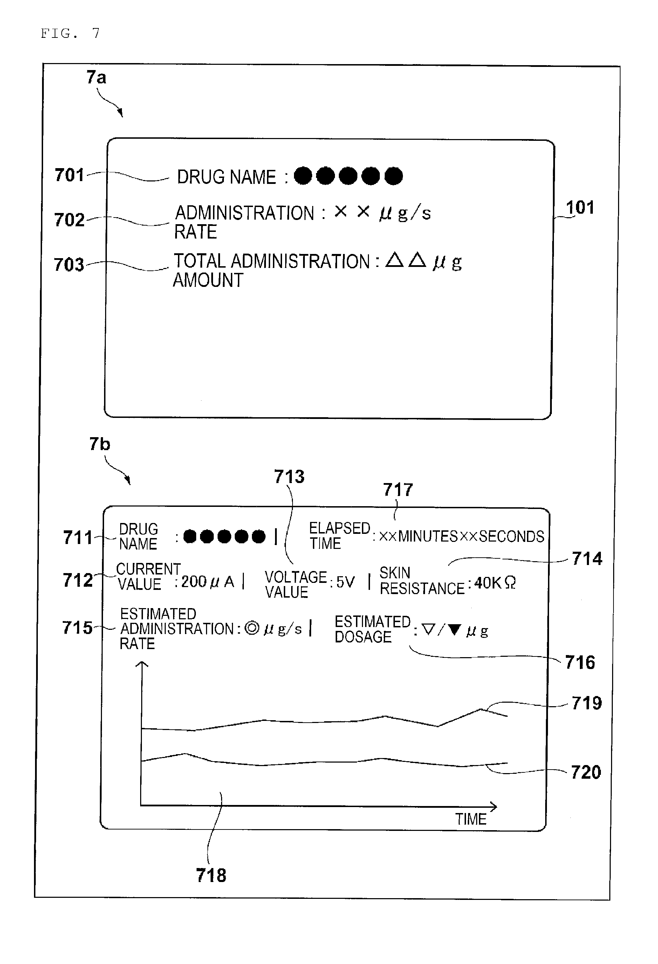

The CPU 121 first acquires a parameter indicating relationships between a type of drug (drug name), an administration rate, and a current value (for example, administration rate per 1µA: x(µg/s)/µA, hereinafter, referred to as a rate parameter) from the RF tag chip 205 of the patch 200 via the RF communication unit 122 (Step S601). As illustrated in Fig.7a, for example, the drug name acquired here is displayed on the display 101 as a drug name display 701. The user 501 or the patient 502 (hereinafter, simply referred to as a user) can identify the drug administered by the patch 200 placed on the controller 100. Next, the CPU 121 detects a setting state of the administration rate dial 104, and displays the administration rate instructed by the administration rate dial 104 as an administration rate display 702 (Step S602). If the user operates the administration rate dial 104, the administration rate display is updated accordingly. The administration rate corresponding to a current value varies depending on a type of drug. Thus, the CPU 121 calculates a current value corresponding to the designated administration rate by using the rate parameter acquired from the patch 200 in Step S601.

Then, the CPU 121 detects a setting state set by the total administration amount dial 105, and acquires a total administration amount instructed by an operator (Step S603). The acquired total administration amount is displayed as a total administration amount display 703. Thereafter, the CPU 121 waits for the administration control switch 103 to be turned on (Step S604). While the administration control switch 103 is turned off, the processes are repeated from Step S601 to Step S604. If the administration control switch 103 is turned on, the process proceeds from Step S604 to Step S605. As the administration rate and the total administration amount, a value set at the time when the process proceeds to Step S605 is adopted.

While the processes are repeated from Step S601 to Step S604, the CPU 121 causes the green lamp 107 to blink so as to show that the administration rate or the total administration amount is being set. Then, if the administration control switch 103 is turned on and the process proceeds to Step S605, the CPU 121 causes the green lamp 107 to be continuously lit so as to inform that the drug is being administered.

If the administration control switch 103 is turned on, the CPU 121 starts to administer the drug held in the patch 200. The CPU 121 first actuates the timer 123, and measures a time elapsed from the start of administration (Step S605). Then, the CPU 121 instructs the driver 113 so as to supply a current value corresponding to the administration rate set in Step S602. The driver 113 includes a constant current source for supplying a current according to the instructed current value, and applies a required voltage to the connecting hooks 191 and 192. In addition, at this timing, the CPU 121 causes the display 101 to switch display content on an administration display screen as illustrated in Fig.7b, and causes the display 101 to display the drug name acquired at the time when the process proceeds to Step S605 as a drug name display 711.

The CPU 121 judges whether or not the drug administration is completed. For example, whether the drug administration is completed can be judged by judging whether an administration amount estimated through an estimation process (to be described later) of the drug (Step S612) reaches a total administration amount designated in Step S603. Alternatively, a required administration time may be calculated by dividing the total administration amount set in Step S603 by the administration rate set in Step S602. In this manner, when a time set based on this calculation elapses, it may be judged that the administration is completed. If it is judged that the drug administration is completed, the process proceeds to Step S609, and this process is completed after stopping voltage application for administering the drug.

On the other hand, if the administration is not completed, the process proceeds to Step S608, and it is judged whether it is the time for sampling. If it is not the time for sampling, the process returns to Step S607. On the other hand, if it is the time for sampling, the process proceeds to Step S610. The time for sampling is set to occur at a predetermined time interval, for example, every second.

If it is judged as the timing for sampling, the CPU 121 acquires a voltage value and a current value between the connecting hooks 191 and 192, that is, between electrodes, from the voltage/current detection unit 114 (Step S610). Then, the CPU 121 calculates an estimated administration rate by using the parameter acquired in Step S601 and the current value acquired in Step S610 (Step S611). Furthermore, the CPU 121 estimates an administered dosage at the present time, based on an administration rate from the start of administration to the present time (Step S612).

Then, the CPU 121 displays a voltage value and a current value which are acquired in Step S610, and a skin resistance value calculated therefrom, as a current value display 712, a voltage value display 713, and a skin resistance display 714 (Step S613, refer to Fig.7b). In addition, the CPU 121 causes the display 101 to display an administration rate (estimated administration rate) calculated in Step S611, as an estimated administration rate display 715 (Step S613). In addition, the CPU 121 causes the display 101 to display the administered dosage which is estimated in Step S612, as an estimated dosage display 716 (Step S613). In the present embodiment, the estimated dosage display 716 is performed by using an expression of (estimated dosage up to the present time) / (total administration amount). In addition, the CPU 121 acquires an elapsed time from the start of administration through the timer 123, and causes the display 101 to display the elapsed time as an elapsed time display 717.

Furthermore, the CPU 121 displays a time-dependent change in the voltage value and the current value from the start of administration, as a graph 718. In an example illustrated in Fig. 7b, the graph 718 displays a time-dependent change 719 in the voltage value and a time-dependent change 720 in the current value. During the process of Step S607 to Step S614, the CPU 121 draws a user's attention by causing the red lamp 106 to blink when the detected voltage value reaches the maximum limit value.

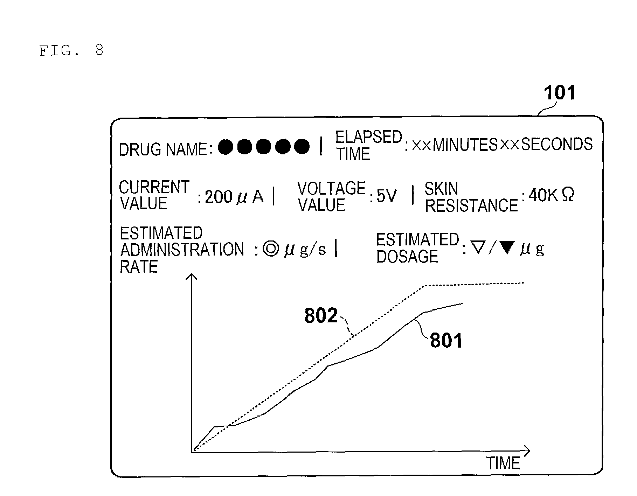

As long as the time-dependent change relates to the drug administration, any display such as an administration rate, an administered dosage (estimated dosage), and the like may be performed as the graph display. For example, when a time-dependent change in the estimated dosage is to be displayed, it may be considered to employ a display form as illustrated in Fig. 8. Here, the time-dependent change in the estimated dosage is displayed as a time-dependent change 801. In this case, if a time-dependent change in an ideal dosage obtained from the administration rate set in Step S602 and the total administration amount set in Step S603 are displayed as illustrated by a dotted line 802, an operator can recognize a relationship between a setting state and an actual administration amount at a glance. In addition, a configuration may be adopted in which the displays 712 to 717 or various graphs are switched to one another.

A configuration has been described in which the administration rate and the total administration amount of the drug are manually set. However, as disclosed in PTL 2, a current profile or the like may be acquired from the cradle 300, and an application voltage may be controlled according to the current profile. In this case, for example, as the display of the dotted line 802 in Fig. 8, a time-dependent change in a dosage according to the profile acquired from the cradle 300 may be displayed.

In addition, a parameter indicating a relationship between the administration rate and the current value is acquired by means of RF communication with the patch 200. However, a configuration is not limited thereto. For example, a table in which a "type of drug" and a "parameter indicating the relationship between the administration rate and the current value" are associated with each other may be held in the internal memory 124, and a parameter may be acquired with reference to the table. In this case, the type of drug (drug name) may be acquired by means of the RF communication with the patch 200, or for example, an operator may manually designate the type of drug by disposing a drug designation dial. Furthermore, the controller 100 may read a pin arrangement or the like of the patch 200, and may detect the type of drug.

In addition, when the drug administration device according to the present invention adopts a configuration in which the drug administration device is portable and the overall device is used by sticking to a patient's skin, the patient can easily confirm a state of drug administration by himself or by herself. Therefore, the patient can be provided with a sense of relief.

The present invention is not limited to the above-described embodiment, and can be changed and modified in various ways without departing from the spirit and the scope of the present invention. Therefore, the following claims are appended herein in order to announce the scope of the present invention publically.

## Claims

1. A drug administration device which applies a voltage between electrodes so as to percutaneously administer a drug, the drug administration device comprising:
measurement means for measuring a current value between the electrodes at predetermined time intervals;
calculation means for calculating an administration rate of the drug based on the current value measured by the measurement means; and
control means for causing a display to display the administration rate calculated by the calculation means.

2. The drug administration device according to Claim 1, further comprising:
estimation means for estimating a dosage administered from when the drug administration starts up to the present time, based on the administration rate calculated by the calculation means,
wherein the control means causes the display to display the dosage estimated by the estimation means.

3. The drug administration device according to Claim 2, further comprising:
stop means for stopping voltage application between the electrodes if the dosage estimated by the estimation means reaches a dosage designated by an operator.

4. The drug administration device according to any one of Claims 1 to 3,
wherein the measurement means further measures a voltage value between the electrodes at the predetermined time intervals, and
wherein the control means further causes the display to display at least any one of the current value and the voltage value which are measured by the measurement means, and a skin resistance value obtained from the current value and the voltage value.

5. The drug administration device according to Claim 4,
wherein the control means uses a graph so as to display a time-dependent change in any one of the current value and the voltage value which are measured at the predetermined time intervals, the skin resistance value obtained from the current value and the voltage value, and the administration rate calculated by the calculation means.

6. The drug administration device according to Claim 2,
wherein the control means uses a graph so as to display a time-dependent change in the dosage estimated by the estimation means.

7. The drug administration device according to any one of Claims 1 to 6, further comprising:
communication means for communicating with a patch placed thereon while being configured to have the patch which holds a drug and is freely detachable.

8. The drug administration device according to Claim 7,
wherein the control means acquires the name of the drug held by the patch via the communication means, and causes the display to display the name of the drug.

9. A control method of a drug administration device which applies a voltage between electrodes so as to percutaneously administer a drug, the control method comprising:
a measurement step of measuring a current value between the electrodes at predetermined time intervals;
a calculation step of calculating an administration rate of the drug, based on the current value measured in the measurement step; and
a control step of causing a display to display the administration rate calculated in the calculation step.
